# EUROPEAN PATENT APPLICATION

(11) **EP 1 611 906 A1**
(43) Date of publication of application: **04.01.2006**
(21) Application number: 04396041.8
(22) Date of filing: 30.06.2004
(51) Int. Cl.: A61L 27/10, A61L 27/34, A61L 27/54, A61L 31/02, A61L 31/10, A61L 31/16, B32B 17/02, B32B 17/06

(54) **A multilayer material**

(71) Applicant: Yli-Urpo, Antti, 20660 Littoinen (FI); Ylänen, Heimo, 20810 Turku (FI); Korventausta, Joni, 20540 Turku (FI); Arstila, Hanna, 20100 Turku (FI); Närhi, Timo, 00340 Helsinki (FI); Hupa, Mikko, 20720 Turku (FI); Vallittu, Pekka, 21620 Kuusisto (FI)
(72) Inventor: Yli-Urpo, Antti, 20660 Littoinen (FI); Ylänen, Heimo, 20810 Turku (FI); Korventausta, Joni, 20540 Turku (FI); Arstila, Hanna, 20100 Turku (FI); Närhi, Timo, 00340 Helsinki (FI); Hupa, Mikko, 20720 Turku (FI); Vallittu, Pekka, 21620 Kuusisto (FI)
(74) Representative: Suominen, Kaisa Liisa

(57) **Abstract**

The invention relates to a multilayer material comprising at least two layers of web and at least one layer of particles, said particles being arranged between said at least two layers of web. According to the invention at least a part of said multilayer material is made of bioactive glass. The invention also relates to different uses of said multilayer material.

## Description

### FIELD OF THE INVENTION

This invention relates to a multilayer material comprising at least two layers of web and at least one layer of particles, said particles being arranged between said at least two layers of web. The invention also relates to different uses of said multilayer material.

### BACKGROUND OF THE INVENTION

The publications and other materials used herein to illuminate the background of the invention, and in particular, the cases to provide additional details respecting the practice, are incorporated by reference.

The use of bioactive glasses in medicine are now widely know. In this application, by bioactive glass is meant a material that has been designed to induce specific biological activity in body tissue. The term biodegradable in this context means that it is degradable upon prolonged implantation when inserted into the mammal body. By biomaterial a non-viable material used in a medical device is meant, a material that is intended to interact with biological systems.

Bioactive glasses react in aqueous systems and develop layers on their surfaces resulting in bonding between the device and the host tissue. Unlike most other bioactive materials, the rate of chemical reactions of bioactive glasses can be easily controlled by changing the chemical composition of the glass. Therefore, bioactive glasses are interesting in particular in clinical applications and have indeed been used for example to treat cranio-facial injuries, to replace the small bones (ossicles) in the middle ear and in orthopaedic surgery to fill defects in bone.

The document US 6,743,513 discloses a tape cast multilayer ceramic and metal composite. In this material, a bioactive layer is first cast on a tape and the metal layers, or thin metal foils, are then laminated on the bioactive layer. For casting of the bioactive layer, fine particles of bioactive glass, organic binder as well as plasticizers and binders are mixed to form a homogeneous slurry which is then cast on a tape. The organic compounds are then removed before sintering of the material.

### OBJECTS AND SUMMARY OF THE INVENTION

The object of this invention is to provide a material suitable for use as a bioactivity enhancing material that has also sufficient structural strength and stability.

A further object of this invention is to provide a material that may be used for tissue *culture in vitro* and that allows the transfer of the tissue without damaging it.

The object is also to provide a material that is useful in the treatment of gingivitis. In addition, the object of the present invention is to provide a material that is easy and convenient to use.

The present invention relates to a multilayer material comprising at least two layers of web and at least one layer of particles, said particles being arranged between said at least two layers of web. The invention is typically characterised in that at least a part of said multilayer material is made of bioactive glass.

The present invention also relates to various uses of said multilayer material.

### DETAILED DESCRIPTION OF THE INVENTION

The invention is disclosed in the appended independent claims.

The multilayer material according to the invention comprises at least two layers of web and at least one layer of particles, said particles being arranged between said at least two layers of web. The invention is typically characterised in that at least a part of said multilayer material is made of bioactive glass.

Thus, the invention concerns a material comprising at least two structural layers made of web and at least one intermediate layer made of particles that hold the two layers of web at a distance from each other. The present invention thus provides a material suitable for use as a bioactivity enhancing material that has also sufficient structural strength and stability.

According to the invention, the multilayer material may comprise two, three, four, five, six, seven or more layers of web. By web, it is meant materials such as woven tissues, nonwoven tissues or veils. The material thus has, on a microscopic level, a net-like structure. By net-like structure, it is to be understood both uniform and non-uniform net structures.

The multilayer material may also comprise one, two, three, four, five, six or more layers of particles. Within a layer, the particles are preferably arranged at a distance from each other. Also, according to a preferred embodiment of the invention, one layer of particles is arranged between each two layers of web, in order to keep them at a distance from each other. The particles thus act as "space-keepers" in the multilayer material.

According to another embodiment of the present invention, the particles are selected from the group consisting of sol-gel derived silica particles, sol-gel derived titanium oxide particles, bioactive glass particles, particles made of a sintered mixture of hydroxyl apatite and bioactive glass as well as mixtures thereof. The particles, particularly the sol-gel derived particles may comprise silica (silicium oxide) and/or titanium oxide up to 100 mol-%, calcium up to 50 mol-%, phosphorous up to 15 mol-%, proteins or other therapeutically active agents up to 20 mol-%. The particles may also consist of mixtures of these different materials. When hydroxyl apatite is used, it may be for example in the form of powder or granules and it may be combined with calcium phosphate.

The particles may be in the form of spheres or particles having a more non-uniform shape. The particles may also be in the form of very short fibers. The diameter of the particles is preferably 10-1000 µm.

The degradation rate of the particles may vary. For example, part of the particles may degrade and/or dissolve at a high rate, such as within a week when placed within the body of a mammal. According to an embodiment of the invention, the degradation rate of at least a part of said particles is higher than the degradation rate of the webs. It is naturally also possible to use webs having different rates of degradation as well as to use webs comprising different fibers having different rates of degradation.

The material according to the present invention may be deformed in certain limits. The bending or folding of the material may be used to induce zones of different activity, since the force (compression or elongation) increases the potential at the surface of the material and thus creates polarities. These polarities are then used to enhance the growth of the tissue.

According to yet another embodiment of the invention, at least a part of said particles may comprise an agent selected from the group consisting of therapeutically active agents, proteins and mixtures thereof. The multilayer material may thus be used for delivery of different agents or the said agents may be incorporated in the material to induce a specific reaction beneficial for example for the attaching of the material to body tissue. It is also possible to use proteins that enhance the tissue growth.

Some examples of therapeutically active agents useful in the present invention are heparin, antibiotics, anti-inflammatory agents, growth factors, other proteins, stem cells, cancer drugs. The therapeutically active agents may be used for systemic or local application.

According to an embodiment of the invention, the layers of web are made of fibers selected from the group consisting of bioactive glass fibers, E-glass fibers, carbon fibers, aramid fibers, polyethylene fibers, polypropylene fibers and mixtures thereof. Any other known fibers may also be used. Preferably the fibers are biocompatible. The diameter of said fibers is typically 1 µm - 1000 µm, for bioactive glass fibers typically 1 µm - 200 µm. The fibers may also consist of several layers, for instance having several different coating layers.

According to an embodiment of the invention, different layers of web may be made of different fibers. Also, it is possible that one web comprises two or more different kinds of fibers. For example, for tissue guiding use, the nature of the fibers and their location is selected so as to induce a precisely localized degradation and thus tissue growth in a predetermined direction.

According to another embodiment of the invention, the fibers are at least partly coated with a coating selected from the group consisting of polymeric coating, sol-gel derived silica coating, sol-gel derived titanium dioxide coating and mixtures thereof. The coating may be biodegradable or non-biodegradable. The coating may also be doped with calcium and/or phosphate.

The polymeric coating may be for example a coating that binds the fibers together and/or binds the fibers to the particles. Examples of such polymers are acrylates, derivatives thereof, polylactides, ε-caprolactone, polylactic acid, polyglycolic acid, silanes, copolymers and mixtures thereof. The polymers may be bioactive or bioinerts. A sol-gel derived silica coating is typically used when it is desired to further enhance the bioactivity of the multilayer material. A sol-gel derived titanium dioxide coating is used for example when it is desired to obtain a good contact and adhesion with soft tissues. The different layers of web may be coated differently and one layer may comprise different coatings at different locations or sides of the web. It is naturally also possible to coat only a part of said webs or all of them fully.

One bioactive glass composition that is useful in the present invention comprises SiO₂, Na₂O, CaO, K₂O, MgO, P₂O₅ and B₂O₃, wherein the amount of
SiO₂ is 51-56 wt-% of the starting oxides,
Na₂O is 7-9 wt-% of the starting oxides,
CaO is 21-23 wt-% of the starting oxides,
K₂O is 10-12 wt-% of the starting oxides,
MgO is 1-4 wt-% of the starting oxides,
P₂O₅ is 0,5-1,5 wt-% of the starting oxides, and
B₂O₃ is 0-1 wt-% of the starting oxides,
provided that the total amount of Na₂O and K₂O is 17-20 wt-% of the starting oxides. This is called composition A in this specification.

Another type of suitable bioactive glass composition is disclosed in WO 96/21628. A typical composition of these glasses is

| | |
|---|---|
| SiO₂ | 53 - 60 wt-% of the starting oxides, |
| Na₂O | 0 - 34 wt-% of the starting oxides, |
| K₂O | 1 - 20 wt-% of the starting oxides, |
| MgO | 0 - 5 wt-% of the starting oxides, |
| CaO | 5 - 25 wt-% of the starting oxides, |
| B₂O₃ | 0 - 4 wt-% of the starting oxides and |
| P₂O₅ | 0.5 - 6 wt-% of the starting oxides, |

provided that
Na₂O + K₂O = 16 - 35 wt-% of the starting oxides,
K₂O + MgO = 5 - 20 wt-% of the starting oxides and
MgO + CaO = 10 - 25 wt-% of the starting oxides.

According to one embodiment, the fibers are made of a bioactive glass having the following composition: Na₂O 6- wt-% of the starting oxides, K₂O 12 wt-% of the starting oxides, MgO 5 wt-% of the starting oxides, CaO 20 wt-% of the starting oxides, P₂O₅ 4 wt-% of the starting oxides and SiO₂ 53 wt-% of the starting oxides. Another suitable bioactive glass composition is Na₂O 6 wt-% of the starting oxides, K₂O 12 wt-% of the starting oxides, MgO 5 wt-% of the starting oxides, CaO 15 wt-% of the starting oxides, P₂O₅ 4 wt-% of the starting oxides and SiO₂ 58 wt-% of the starting oxides.

In the compositions above, the amount of different oxides is given as weight percent of the starting oxides because some elements, such as sodium, evaporate during the heating. The amounts of the final oxides are however close to those of the starting oxides and in any case, the difference between the starting amounts and the final amounts is less than 5 percentage units, preferably less than 3 percentage units.

It is obvious to a person skilled in the art that the amounts of the oxides can be freely chosen within the above-mentioned limits. Indeed, the amount of SiO₂ can be for example 51,5, 52, 53,5, 55 or 56 wt-% of the starting oxides, the amount of Na₂O can be for example 7, 7,3, 7,7, 8, 8,5 or 9 wt-% of the starting oxides, the amount of CaO can be for example 21, 21,4, 21,7, 22, 22,6 or 23 wt-% of the starting oxides, the amount of K₂O can be for example 10, 10,5, 10,6, 11, 11,3, 11,7 or 12 wt-% of the starting oxides, the amount of MgO can be for example 1, 1,3, 1,9, 2,4, 2,7, 3,5 or 4 wt-% of the starting oxides, the amount of P₂O₅ can be for example 0,5, 0,7, 1, 1,2 or 1,5 wt-% of the starting oxides, and the amount of B₂O₃ can be for example 0, 0,4, 0,6, 0,9 or 1 wt-% of the starting oxides.

When the composition A is used, according to an embodiment of the invention, the amount of SiO₂ is 54-56 wt-% of the starting oxides.

According to another embodiment of the invention in relation to composition A, the glass composition further comprises Al₂O₃ up to 1 wt-% of the starting oxides provided that the total amount of B₂O₃ and Al₂O₃ is 0,5-2,5 wt-% of the starting oxides.

According to yet another embodiment of the invention in connection to composition A, the decrease in the amount of Na₂O and/or K₂O is compensated by the increase of the amount of Al₂O₃ and/or B₂O₃.

The bioactive glass having the kind of composition A may be processed with any conventional methods. A particularly preferred method for the treatment is heating with laser since it allows localized yet high temperatures to be used in the melting of the glass.

The glass composition A that may be used in the present invention is advantageously prepared in atmospheric pressure and at temperatures of about 1360 °C. The heating time for making the glass melt is typically three hours. No protection gas is needed. When preparing the glass composition A, the constituents are first melted together and then cooled down. The resulting solid material is then crushed and remelted in order to obtain a homogeneous material.

The present glass composition A may advantageously be used in the form of fibres. Indeed, the composition A may be drawn to a fibre at higher temperatures than other known bioactive glass compositions. Typically, the manufacturing temperature may be even 100 °C higher than for the conventional bioactive glass compositions. Higher manufacturing temperatures lead to fibres having a smaller diameter since the viscosity of the glass melt decreases with increasing temperature. Also, the manufacturing temperature is critical for the resulting fibre product since it is close to the softening temperature of the glass, thus close to the crystallization temperature. A fibre manufactured from the present composition A that has been heat-treated three times still has the described properties.

The bioactive glass composition A useful in the present invention is disclosed in the application EP 02079105.9, the contents of which are incorporated by reference herein.

The material according to the present invention may for example be in the form of a sheet, a tissue, a tube such as a stent, a ring or a band. The different forms may be obtained by using webs that are already in the desired form, such as a woven tube, or by forming them starting from a sheet, by any technique known *per se.*

The stiffness and strength of the multilayer material depends on the nature of the materials used for webs and particles as well as on the diameter of the fibers and the attaching of the fibers to the particles. The stiffness may be designed from highly stiff to easily foldable. Preferably, the structure is elastic, i.e. it can be deformed to at least a certain extent. The multilayer material according to the present invention may also be sewable, i.e. it may be possible to sew the different layers of web to each other, to form a tube by rolling the multilayer material and then sew it into shape, or to sew the multilayer material directly onto a body tissue.

Also the thickness of the multilayer material as well as its weight per surface unit may vary depending on the application.

The fibers of the web may be attached to the particles by sintering, especially when glass materials are used. Sintering may also be used to attach the edges of a sheet of multilayer material according to the present invention in order to form a tube. Furthermore, also gluing and other techniques may be used for attaching the layers of web and particles to each other as well as the edges of the material to each other.

The material according to the present invention may have any kind of shape and size. It may for example be used in units as small as in the millimetre range, up to the range of tens of centimetres. It is also possible to manufacture larger devices by combining several smaller parts. The smaller parts may then have different constitutions according to the intended use of the device, which may also be called a functional web.

One method of introducing the present multilayer material in a tube form into the body is to position the material in tube form over a rod, to introduce the rod into the desired location wherein the material attaches to the surrounding tissue, to remove the rod whereby the multilayer material stays in place.

The present invention further relates to different uses of the multilayer material disclosed above. The material may be used for example for delivery of therapeutically active agents, for tissue repair, for coating of an implant, for the manufacture of a device for curing gingivitis as well as for support material for tissue culture.

An example of delivery of therapeutically active agents is a stent made of the present multilayer material in which the inside of the stent comprises heparin and the outside of the stent comprises an agent that enhances the attaching of the stent into the body tissue. The use of the present multilayer material for tissue repair and for tissue guiding has been disclosed above. It is also possible to coat an implant with the material according to the present invention by attaching the material on the surface of the implant by for example sintering or gluing. A device for curing gingivitis is disclosed more in detail below. The present multilayer material is advantageous also as a support material for tissue culture, since once the culture is ready to be transferred, the material offers support during the transfer and may enhance the attaching of the new tissue to the target tissue.

In this specification, except where the context requires otherwise, the words "comprise", "comprises" and "comprising" means "include", "includes" and "including", respectively. That is, when the invention is described or defined as comprising specified features, various embodiments of the same invention may also include additional features. Also, the reference signs should not be construed as limiting the claims.

The invention is described below in greater detail by the following, nonlimiting drawings.

### BRIEF DESCRIPTION OF THE DRAWINGS

Figure 1 illustrates a perspective view of a first embodiment of a multilayer material according to the present invention.
Figure 2 illustrates a side view of a second embodiment of a multilayer material according to the present invention.
Figure 3 illustrates a side view of a third embodiment of a multilayer material according to the present invention.
Figure 4 illustrates a use according to a fourth embodiment of a multilayer material according to the present invention.
Figure 5 illustrates a positioning device of a multilayer material according to a seventh embodiment of the present invention.
Figure 6 illustrates a use according to a sixth embodiment of a multilayer material according to the present invention.

### DETAILLED DESCRIPTION OF THE DRAWINGS

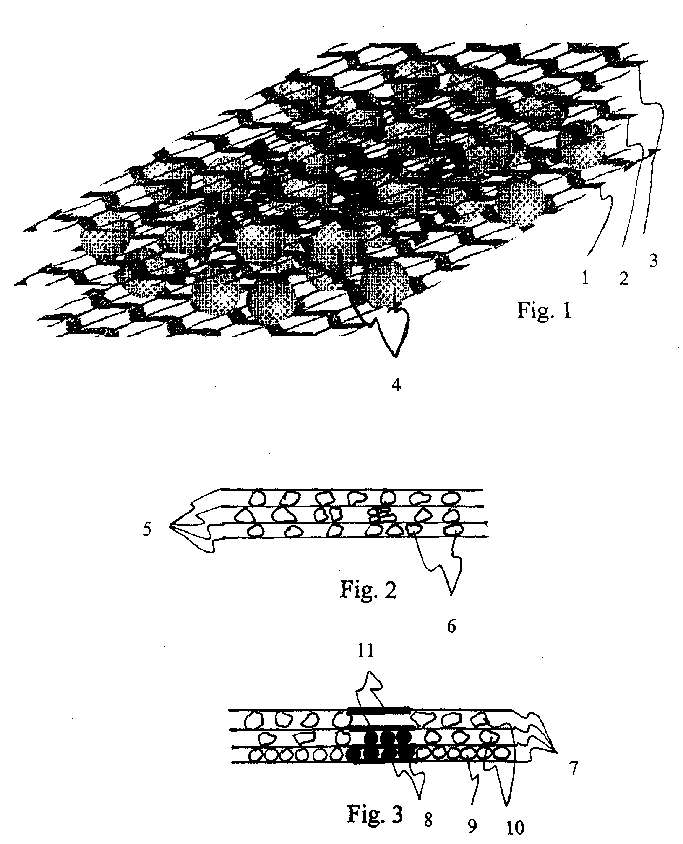

Figure 1 illustrates a perspective view of a first embodiment of a multilayer material according to the present invention. In this embodiment, the material consist of layers 1, 2, 3 of web and of particles 4 positioned between the layers 1, 2 and 3. The layers 1, 2, 3 may be made of same or different materials and each layer may comprise one or more constituents.

Figure 2 illustrates a side view of a second embodiment of a multilayer material according to the present invention. In this second embodiment, the layers 5 are spaced apart in such a way that the distance from one layer to another layer is substantially equal on the total thickness of the material. The diameter of the particles 6 varies and some of the particles are essentially regular in shape, for example spheres, and some of the particles are of a more irregular shape.

Figure 3 illustrates a side view of a third embodiment of a multilayer material according to the present invention. In this embodiment, a tissue guiding material is presented. The layers 7 comprise parts 11 (here illustrated, for the sake of clarity, by thicker lines) that have a faster degradation rate than the rest of the layer. The layers are separated by particles 8, 9 and 10. The particles 8 have a higher degradation rate than the particle 9 and 10, typically essentially the same degradation rate as parts 11. These particles are positioned between the parts 11 of each layer. Particles 9 and 10 are of different type and here also of different shape.

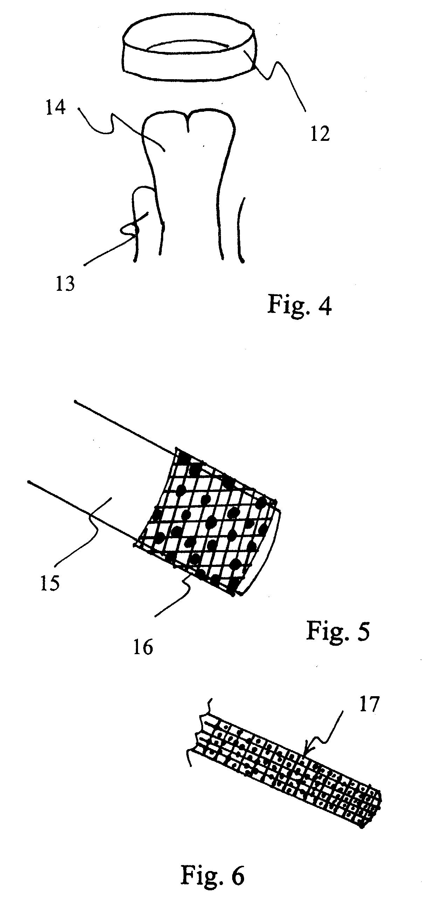

Figure 4 illustrates a use according to a fourth embodiment of a multilayer material according to the present invention. In this embodiment, a device 12 for curing gingivitis has been manufactured from the material according to the present invention. The device 12 consists of a multilayer material in a ring form. The gingival 13 around a tooth 14 is cut open, as illustrated on one side of the tooth 14, the device 12 is positioned around the tooth and the gingival 13 is sewed close. In this embodiment, the multilayer material is preferably fully made of biodegradable materials.

Figure 5 illustrates a positioning device of a multilayer material according to a seventh embodiment of the present invention. The material 16 according to the invention is placed over the positioning device 15, a rod or the like. The positioning device 15 is then inserted into the tissue and as the material 16 reacts with the tissue and becomes attached to it, the positioning device 16 can be removed.

Figure 6 illustrates a use according to a sixth embodiment of a multilayer material according to the present invention. In this embodiment, a stent 17 has been manufactured from the material according to the present invention.

## Claims

1. A multilayer material comprising at least two layers of web and at least one layer of particles, said particles being arranged between said at least two layers of web, **characterized in that** at least a part of said multilayer material is made of bioactive glass.

2. Multilayer material according to claim 1, **characterized in that** said particles are selected from the group consisting of sol-gel derived silica particles, sol-gel derived titanium oxide particles, bioactive glass particles, particles made of a sintered mixture of hydroxyl apatite and bioactive glass as well as mixtures thereof.

3. Multilayer material according to claim 1 or 2, **characterized in that** said layers of web are made of fibers selected from the group consisting of bioactive glass fibers, E-glass fibers, carbon fibers, aramid fibers, polyethylene fibers, polypropylene fibers and mixtures thereof.

4. Multilayer material according to claim 3, **characterized in that** said fibers are at least partly coated with a coating selected from the group consisting of polymeric coating, sol-gel derived silica coating, sol-gel derived titanium dioxide coating and mixtures thereof.

5. Multilayer material according to any of the preceding claims, **characterized in that** the degradation rate of at least a part of said particles is higher than the degradation rate of the webs.

6. Multilayer material according to any of the preceding claims, **characterized in that** at least a part of said particles comprises an agent selected from the group consisting of therapeutically active agents, proteins and mixtures thereof.

7. Use of a multilayer material according to any of the claims 1-6 for delivery of therapeutically active agents.

8. Use of a multilayer material according to any of the claims 1-6 for tissue repair.

9. Use of a multilayer material according to any of the claims 1-6 for coating of an implant.

10. Use of a multilayer material according to any of the claims 1-6 for the manufacture of a device for curing gingivitis.

11. Use of a multilayer material according to any of the claims 1-6 as support material for tissue culture.
